(19) Europäisches Patentamt
European Patent Office
Office européen des brevets

(11) Veröffentlichungsnummer: **0 023 637**
**B1**

(12) # EUROPÄISCHE PATENTSCHRIFT

(45) Veröffentlichungstag der Patentschrift: **04.04.84**

(51) Int. Cl.³: **C 07 D 307/79**, A 01 N 43/12, A 01 N 53/00, C 07 C 69/743

(21) Anmeldenummer: **80104190.6**

(22) Anmeldetag: **17.07.80**

(54) **Substituierte 2,3-Dihydrobenzofurylmethyl-cyclopropancarbonsäure ester, Verfahren zu ihrer Herstellung und Verfahren zur Bekämpfung von Schädlingen und Mittel dafür.**

(30) Priorität: **04.08.79 DE 2931672**

(43) Veröffentlichungstag der Anmeldung: **11.02.81 Patentblatt 81/6**

(45) Bekanntmachung des Hinweises auf die Patenterteilung: **04.04.84 Patentblatt 84/14**

(84) Benannte Vertragsstaaten: **AT BE CH DE FR GB IT LI NL SE**

(56) Entgegenhaltungen:
**DE - A - 2 108 932**
**DE - A - 2 255 581**

(73) Patentinhaber: **BASF Aktiengesellschaft, Carl-Bosch-Strasse 38, D-6700 Ludwigshafen (DE)**

(72) Erfinder: **Schwarz, Gerd-Ulrich, Dr., Danziger Strasse 10, D-6800 Mannheim 71 (DE)**
Erfinder: **Kiehs, Karl, Dr., Sudetenstrasse 22, D-6840 Lampertheim (DE)**
Erfinder: **Boell, Walter, Dr., Hintergasse 35, D-6701 Dannstadt-Schauernheim 1 (DE)**
Erfinder: **Adolphi, Heinrich, Dr., Kalmitweg 11, D-6701 Limburgerhof (DE)**

EP 0 023 637 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Substituierte 2,3-Dihydrobenzofurylmethyl-cyclopropancarbonsäureester, Verfahren zu ihrer Herstellung und Verfahren zur Bekämpfung von Schädlingen und Mittel dafür

Gegenstand der Erfindung sind substituierte 2,3-Dihydro-benzofurylmethyl-cyclopropancarbonsäureester, ein Verfahren zu ihrer Herstellung, Schädlingsbekämpfungsmittel, die diese Ester als Wirkstoffe enthalten, sowie ein Verfahren zur Bekämpfung von Schädlingen mit diesen Wirkstoffen.

Es ist bekannt, daß 2,2-Dimethyl-3-(2',2'-dimethylvinyl)-cyclopropancarbonsäureester, die als Alkoholkomponenten einen substituierten 2-Methyl-2,3-dihydrobenzofuranrest enthalten, insektizidwirksam sind (DE-A-2 108 932 und DE-A-2 255 581).

Es wurde gefunden, daß substituierte 2,3-Dihydrobenzofurylmethyl-cyclopropancarbonsäureester der Formel (I)

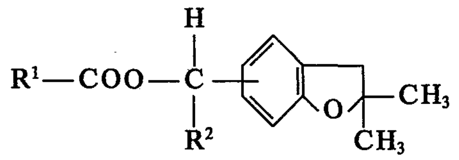

(I)

in der

$R^1$ für den 3-(2,2-Dichlorvinyl)- oder den 3-(2,2-Dibromvinyl)-2,2-dimethyl-cyclopropylrest und
$R^2$ für Wasserstoff, Alkyl oder Alkenyl mit jeweils bis zu 5 Kohlenstoffatomen stehen,

sich sehr gut zur Bekämpfung von Schädlingen, insbesondere von Insekten, Milben und Zecken, eignen.

Beispiele für unverzweigte oder verzweige Alkyl- oder Alkenylgruppen mit bis zu 5 Kohlenstoffatomen für $R^2$ sind Methyl, Äthyl, n-Propyl, Isopropyl, n-Butyl, tert.-Butyl, n-Pentyl, Isopentyl, Vinyl, Allyl, n-Propen-1-yl, Isopropenyl, n-Butenylreste, 2-Methyl-propen-1-yl, n-Pentenylreste, 1-Methyl-n-propenylreste und 1-Methyl-n-butenylreste.

Bevorzugte Verbindungen der Formel (I) sind solche, bei denen $R^2$ für Wasserstoff, Isopropyl oder Vinyl steht.

Beispiele für erfindungsgemäße 2,3-Dihydrobenzofurylmethylester der Formel I sind

2,2-Dimethyl-3-(2',2'-dichlorvinyl)-cyclopropancarbonsäure[2'',2''-dimethyl-2'',3''-dihydrobenzofuran-6''-methyl-(α-vinyl)-ester,
2,2-Dimethyl-3-(2',2'-dichlorvinyl)-cyclopropancarbonsäure-[2'',2''-dimethyl-2'',3''-dihydrobenzofuran-6-methyl-(α-isopropyl)]-ester,
2,2-Dimethyl-3-(2',2'-dichlorvinyl)-cyclopropancarbonsäure-(2',2'-dimethyl-2'',3''-dihydrobenzofuran-6''-methyl)-ester,
2,2-Dimethyl-3-(2',2'-dichlorvinyl)-cyclopropancarbonsäure-[2'',2''-dimethyl-2'',3''-dihydrobenzofuran-6''-methyl-(α-allyl)]-ester,
2,2-Dimethyl-3-(2',2'-dibromvinyl)-cyclopropancarbonsäure-[2'',2''-dimethyl-2'',3''-dihydrobenzofuran-6''-methyl]-ester,
2,2-Dimethyl-3-(2',2'-dibromvinyl)-cyclopropancarbonsäure-[2'',2''-dimethyl-2'',3''-dihydrobenzofuran-6''-methyl-(α)vinyl)]-ester,
2,2-Dimethyl-3-(2',2'-dibromvinyl)-cyclopropancarbonsäure-[2'',2''-dimethyl-2'',3''-dihydrobenzofuran-6''-methyl-(α-allyl)]-ester,
2,2-Dimethyl-3-(2',2'-dibromvinyl)-cyclopropancarbonsäure-[2'',2''-dimethyl-2'',3''-dihydrobenzofuran-6''-methyl-(α-isopropyl)]-ester,
2,2-Dimethyl-3(2',2'-dichlorvinyl)-cyclopropancarbonsäure[2'',2''-dimethyl-2'',3''-dihydrobenzofuran-4''-methyl-(α-allyl)]ester,
2,2-Dimethyl-3-(2',2'-dibromvinyl)-cyclopropancarbonsäure-[2'',2''-dimethyl-2'',3''-dihydrobenzofuran-4''-methyl]-ester,
2,2-Dimethyl-3-(2',2'-dibromvinyl)-cyclopropancarbonsäure-[2'',2''-dimethyl-2'',3''-dihydrobenzofuran-4''-methyl-(α-vinyl)]-ester,
2,2-Dimethyl-3-(2',2'-dibromvinyl)-cyclopropancarbonsäure-[2'',2''-dimethyl-2'',3''-dihydrobenzofuran-4''-methyl-(α-allyl)]-ester,
2,2-Dimethyl-3-(2',2'-dibromvinyl)-cyclopropancarbonsäure-[2'',2''-dimethyl-2'',3''-dihydrobenzofuran-4''-methyl-(α-isopropyl)]-ester,
2,2-Dimethyl-3-(2',2'-dichlorvinyl)-cyclopropancarbonsäure[2'',3''-dimethyl-2'',3''-dihydrobenzofuran-6''-methyl-(α-allyl)]-ester,
2,2-Dimethyl-3-(2',2'-dibromvinyl)-cyclopropancarbonsäure-[2'',3''-dihydrobenzofuran-6''-methyl-(α-isopropyl)]-ester.

Es versteht sich von selbst, daß die Bezeichnung der Ester sämtliche mögliche Isomere umfaßt.

Es wurde weiterhin gefunden, daß die 2,3-Dihydro-benzofurylmethylester der Formel I durch Umsetzung von Säurehalogeniden der Formel (II)

$R^1-CO-Hal$ (II)

in der $R^1$ die obengenannte Bedeutung hat und Hal für Halogen, insbesondere für Chlor, steht, mit Verbindungen der Formel (III)

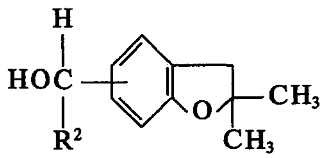

(III)

in der $R^2$ die obengenannten Bedeutungen hat, in Gegenwart eines säurebindenden Mittels erhalten werden.

Darüber hinaus ist es möglich, die neuen Ester der Formel (I) durch Umsetzung von Säuren der Formel (IV)

$R^1-COOH$ (IV)

in der $R^1$ die obengenannten Bedeutungen hat, mit Halogeniden der Formel V

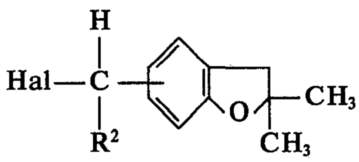

(V)

in der $R^2$ die obengenannten Bedeutungen hat und Hal für Halogen, insbesondere Chlor, steht, in Gegenwart eines säurebindenden Mittels herzustellen.

Weitere Synthesewege sind die Umsetzung von Säuren der Formel (IV) mit Verbindungen der Formel (III) in Gegenwart eines wasserbindenden Mittels und die Umsetzung von Alkylestern der Formel (VI)

$R^1-COOR$ (VI)

in der $R^1$ die obengenannten Bedeutungen hat und R für unverzweigtes oder verzweigtes Alkyl mit 1 bis 5 Kohlenstoffatomen steht, mit Verbindungen der Formel (III) in Gegenwart eines für Umesterungen üblichen Katalysators.

Die einzelnen Reaktionswege lassen sich durch folgende Reaktionsgleichungen darstellen:

a) $R^1—CO—Hal$ +

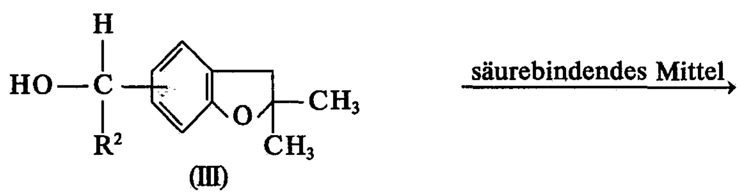


(I)

(II) (III)

b) $R^1—COOH$ +

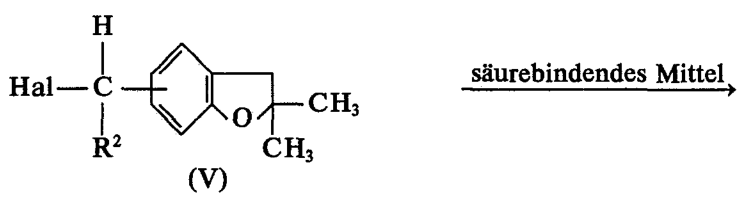


(I)

(IV) (V)

c) $R^1—COOH$ +

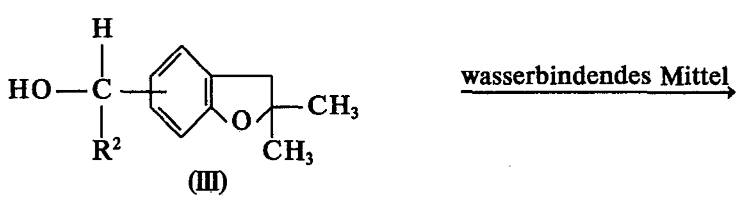


(I)

(IV) (III)

d) $R^1$—COOR (VI) + 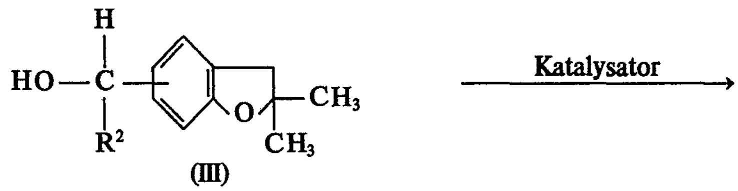

(III) (I)

Als säurebindendes Mittel für die Synthesewege a) und b) kommen organische Basen, beispielsweise tertiäre Amine, wie Triäthylamin, Trimethylamin, Dimethylanilin, Dimethylbenzylamin, 4-Dimethylamino-pyridin, 4-Pyrrolidinopyridin oder Pyridin, sowie anorganische Basen, wie Hydroxide, Oxide, Hydrogencarbonate oder Carbonate von Alkali- oder Erdalkalimetallen, beispielsweise Natriumhydroxid, Kaliumhydroxid, Calciumhydroxid, Calciumhydrogencarbonat, Kaliumcarbonat, und Alkoholate oder Hydride der Alkalimetalle, beispielsweise Natriumhydrid oder Kalium-t-butylat, in Betracht. Das säurebindende Mittel wird in mindestens molaren Mengen, bezogen auf die Verbindung der Formel (II) bzw. (V) eingesetzt.

Für die Umsetzung entsprechend Reaktionsgleichung c) eignen sich als wasserbindende Mittel beispielsweise Dicyclohexylcarbodiimid, anorganische Mineralsäuren, wie Schwefelsäure, Phosphorsäure, saure Ionenaustauscher oder p-Toluolsulfonsäure. Das wasserbindende Mittel wird dabei in Mengen von mindestens 0,02, zweckmäßigerweise 0,05 bis 0,4 Mol, bezogen auf 1 Mol der Reaktionskomponente (III) bzw. (IV) verwendet.

Geeignete Katalysatoren für die Umsetzung entsprechend Reaktionsgleichung d) sind Hydride oder Alkoholate der Alkalimetalle, wie Natriumhydrid, Natriumäthylat und Triphenyl-natrium. Ebenso geeignet sind Alkoholate der Elemente der Gruppe IVb des Periodensystems, wie Titantetramethylat, Titantetraäthylat. Pro Mol Ester der Formel (VI) werden zweckmäßigerweise 0,02 bis 0,4 Mol Katalysator zugegeben.

Alle Herstellungsverfahren a) bis d) können bei Reaktionstemperaturen im Bereich zwischen −10 und +150° C, drucklos oder unter Druck, diskontinuierlich oder kontinuierlich durchgeführt werden.

Zur Durchführung der Verfahren setzt man die Reaktionskomponenten der Formeln (II) und (III), (IV) und (V) und (III) sowie (VI) und (III) vorzugsweise in äquimolaren Verhältnissen ein. Ein Überschuß der einen oder anderen Komponente bringt keine wesentlichen Vorteile. Die Umsetzung verläuft praktisch quantitativ.

Die Verfahren a) bis d) werden zweckmäßigerweise in Gegenwart geeigneter Lösungs- oder Verdünnungsmittel durchgeführt. Als solche kommen praktisch alle inerten organischen Solventien in Betracht. Hierzu gehören acyclische und cyclische Äther, wie Diäthyläther, Tetrahydrofuran, Dioxan, alkylierte aliphatische und alicyclische Carbonsäureamide, wie Dimethylformamid, N-Methylpyrrolidon, aliphatische und aromatische, gegebenenfalls chlorierte Kohlenwasserstoffe, wie Toluol, Xylole, Chloroform, n-Hexan, Cyclohexan, Chlorbenzol, Ketone, wie Aceton, Methyläthylketon, Nitrile, wie Acetonitril, ferner Dimethylsulfoxid, Hexamethylphosphorsäuretriamid. Auch Gemische dieser Lösungsmittel können verwendet werden.

Das Verfahren entsprechend Reaktionsgleichung a) kann auch in wäßriger Lösung durchgeführt werden. Als Zweiphasenreaktion können die verfahren a) und b) durchgeführt werden, wobei als wasserunlösliche organische Phase beispielsweise Äther, aliphatische und aromatische, gegebenenfalls chlorierte Kohlenwasserstoffe, insbesondere Toluol oder Chloroform, und Methyläthylketon verwendet werden können.

Werden bei der Herstellung der Ester der Formel (I) nicht einheitliche optisch aktive bzw. cis- oder transisomere Ausgangsmaterialien verwendet, liegen die Endprodukte als Gemische verschiedener optisch aktiver Isomeren sowie von cis/trans-Isomeren vor. Diese Isomerengemische können nach bekannten Methoden in die einheitlichen Isomeren aufgetrennt werden. Unter dem Begriff Ester der Formel (I) versteht man sowohl die reinen Isomeren als auch deren Gemische.

Die Ausgangsverbindungen der Formel (II) sind zum Teil bekannt (GB-A-1 446 304, US-A-3 981 903) oder können analog bekannten Verfahren hergestellt weren. Die Verbindungen der Formel (III) sind zum Teil ebenfalls bekannt (DE-A-2 108 932 und DE-A-2 255 581) oder können nach an sich bekannten Verfahren hergestellt werden, beispielsweise durch Umsetzung von Aldehyden mit Metallorganylen oder durch Anlagerung von Alkinen an Aldehyde mit anschließender partieller Hydrierung (Houben—Weyl, Methoden der organischen Chemie, Bd. V/1b, S. 775 bis 790, Georg Thieme-Verlag, Stuttgart, 1972). Verbindungen der Formel (IV), (V) und (VI) sind ebenfalls bekannt bzw. können nach an sich bekannten Verfahren hergestellt werden. (US-A-3 979 519, US-A-3 981 903, BE-A-801 946, DE-A-2 365 555, DE-A-2 231 312).

Das folgende Beispiel erläutert die Herstellung der erfindungsgemäßen Ester der Formel I,

## Beispiel

73 ml einer 1,6 molaren Lösung von Vinylmagnesiumchlorid in Tetrahydrofuran werden in 200 ml absolutem Tetrahydrofuran bei 0° C vorgelegt. Unter Rühren und Feuchtigkeitsausschluß tropft man

17,6 g 2,2-Dimethyl-2,3-dihydrobenzofuran-6-aldehyd in 100 ml absolutem Tetrahydrofuran zu. Man läßt 3 Stunden bei 25°C rühren und zersetzt das Reaktiongemisch vorsichtig mit einer kalt gesättigten Ammoniumchloridlösung und einigen Millilitern 10%iger Salzsäure, bis sich der Niederschlag auflöst. Dann wird dreimal mit Äther ausgeschüttelt, die vereinigten Ätherextrakte mit Natriumhydrogensulfitlösung, Natriumhydrogencarbonatlösung und Wasser gewaschen, die Natriumsulfat getrocknet und unter vermindertem Druck eingeengt.

Man erhält 17,7 g 2,2-Dimethyl-2,3-dihydrobenzofuran-6-(α-vinyl)-carbinol in Form eines gelblichen Öls, das für die weitere Umsetzung rein genug ist (Ausbeute 87% d. Th.).

8,8 g 2,2-Dimethyl-2,3-dihydrobenzofuran-6-(α-vinyl)-carbinol in 100 ml absolutem Äther und 7 g Pyridin werden bei 0 bis 10°C vorgelegt. Zu dieser Lösung werden 10,9 g 3-(2,2-Dichlorvinyl)-2,2-dimethyl-cyclopropancarbonsäurechlorid in 20 ml absolutem Äther langsam zugetropft. Man läßt über Nacht bei 25°C rühren und saugt danach vom Niederschlag ab. Die Ätherphase wird mit 2 n Salzsäure und 2 n Natriumhydrogencarbonatlösung Wasser gewaschen, über Natriumsulfat getrocknet und eingeengt.

Man erhält (2,2-Dichlorvinyl)-2,2-dimethyl-cyclopropancarbonsäure-(2,2-dimethyl-2,3-dihydrobenzofuran-6-(α-vinyl)-methyl)-ester in Form eines Öls, das über eine Kieselgelsäule mit Toluol als Elutionsmittel gereinigt wird.

$n_D^{21} = 1\ 543$ (Verbindung Nr. 2 gemäß Tabelle).

Analog lassen sich folgende Verbindungen herstellen:

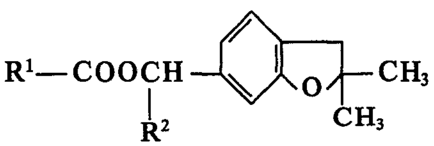

| Nr. | $R^1$ | $R^2$ | $n_D$ |
|---|---|---|---|
| 1 | 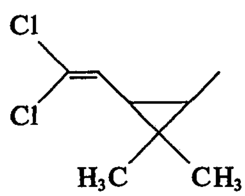 | H | 1.5362 (23°C) |
| 2 | 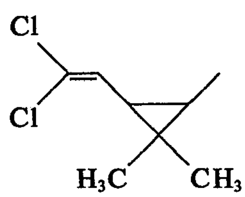 | $CH{=}CH_2$ | 1.543 (21°C) |
| 3 | 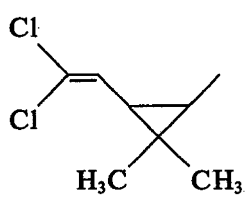 | i-$C_3H_7$ | 1.5337 (21°C) |
| 4 | 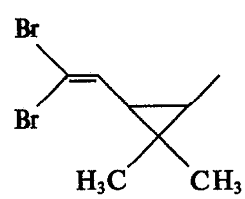 | H | 1.5552 (23°C) |

| Nr. | $R^1$ | $R^2$ | $n_D$/Analyse |
|---|---|---|---|
| 5 | 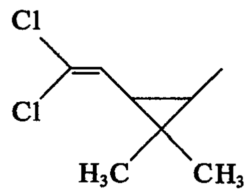 | $CH_3$ | 1.5345 (23°C) |
| 6 | 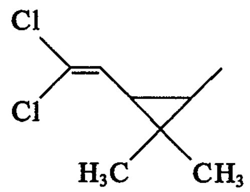 | $C_2H_5$ | 1.5315 (23°C) |
| 7 | 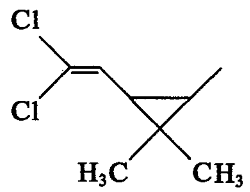 | n-$C_4H_9$ | 1.5240 (24°C) |

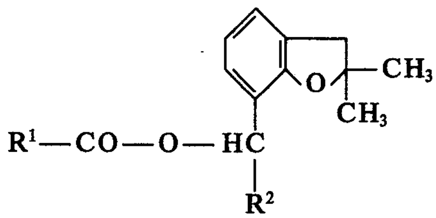

| Nr. | $R^1$ | $R^2$ | $n_D$ |
|---|---|---|---|
| 8 | 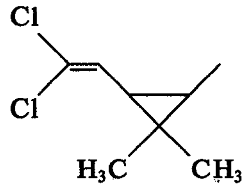 | H | 1.5373 (24°C) |
| 9 | 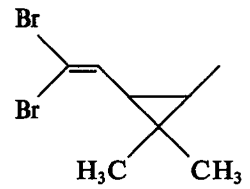 | H | 1.5540 (24°C) |
| 10 | 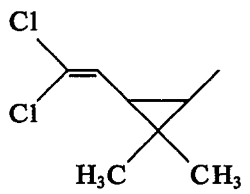 | $CH{=}CH_2$ | 1.5379 (23°C) |
| 11 | 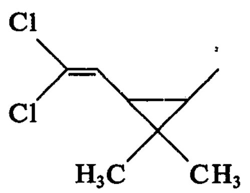 | $i\text{-}C_3H_7$ | 1.5283 (23°C) |
| 12 | 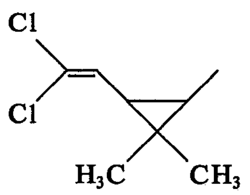 | $n\text{-}C_4H_9$ | 1.5236 (23°C) |

Die erfindungsgemäßen Ester sind geeignet, Schädlinge aus der Klasse der Insekten, Milben und Zecken wirksam zu bekämpfen.

Zu den schädlichen Insekten gehören aus der Ordnung der Schmetterlinge (Lepidoptera) beispielsweise Hepialus humuli (Hopfenspinner), Oncopera fasciculata, Oxycanus cerrinatus, Tinea cloacella (Korkmotte), Tinea pellionella (Pelzmotte), Tineola bisselliella (Kleidermotte), Lyonetia clerkella (Schlangenminiermotte), Plutella maculipennis (Kohlschabe), Leucoptera coeffeella (Kaffeemotte), Hyponomeuta malinellus (Apfelbaumgespinstmotte), Argyresthia conjugella (Apfelmotte), Sitotroga cerealella (Getreidemotte), Phthorimaea operculella (Kartoffelmotte), Capua reticulana (Apfelschalenwickler), Sparganothis pilleriana (Springwurm), Cacoecia murinana (Tannentriebwickler), Tortrix viridana (Eichenwickler), Clysia ambiguella (Heu- und Sauerwurm), Evetria buoliana (Kieferntriebwickler), Polychrosis botrana (Bekreuzter Traubenwickler), Cydia pomonella (Obstmade), Laspeyresia molesta (Pfirsichtriebbohrer), Laspeyresia funebrana (Pflaumenwickler), Ostrinia nubilalis (Maiszünsler), Loxostege sticticalis (Rübenzünsler), Ephestia kuehniella (Mehlmotte), Chilo suppressalis (Reisstengelbohrer), Galleria Mellonella (Wachsmotte), Malacosoma neustria (Ringelspinner), Dendrolimus pini (Kiefernspinner),

Thaumatopoea pityocampa (Pinienprozessionsspinner), Phalera bucephala (Mondfleck), Cheimatobia brumata (Kleiner Frostspanner), Hibernia defoliaria (Großer Frostspanner), Bupalus piniarus (Kiefernspanner), Hyphantria cunea (Weißer Bärenspinner), Agrotis segetum (Wintersaateule), Agrotis ypsilon (Ypsiloneule), Barathra brassicae (Kohleule), Cirphis unipuncta (Heerwurm), Prodenia litura (Baumwollraupe), Laphygma exigua (Rüben-Heerwurm), Panolis flammea (Forleule), Earias insulana (Baumwollkapselwurm), Flusia gamma (Gammaeule), Alabama argil lacea (Baumwollblattwurm), Lymantria dispar (Schwammspinner), Lymantria monacha (Nonne), Pieris brassicae (Kohlweißling), Aporia crataegi (Baumweißling);

aus der Ordnung der Käfer (Coleoptera) beispielsweise Blitophaga undata (Schwarzer Rübenaaskäfer), Melanotus communis (Drahtwurm), Limonius californicus (Drahtwurm), Agriotes lineatus (Saatschnellkäfer), Agricotes obscurus (Humusschnellkäfer), Agrilus sinuatus (Birnbaum-Prachtkäfer), Meligethes aeneus (Rapsglanzkäfer), Atomaria linearis (Moosknopfkäfer), Epilachna varicestris (Mexikanischer Bohnenkäfer), Phyllopertha horticola (Junikäfer), Popillia japonica (Japankäfer), Melolontha melolontha (Feldmaikäfer), Melolontha hippocastani (Waldmaikäfer), Amphimallus solstitialis (Brachkäfer), Crioceris asparagi (Spargelhähnchen), Lema melanopus (Getreidehähnchen), Leptinotarsa decemlineata (Kartoffelkäfer), Phaedon cochleariae (Meerrettich-Blattkäfer), Phyllotreta nemorum (Kohlerdfloh), Chaetocnema tibialis (Rübenflohkäfer), Phylloides chrysocephala (Raps-Flohkäfer), Diabrotica 12-punctata (Südlicher Maiswurzelwurm), Cassida nebulosa (Nebliger Schildkäfer), Bruchus lentis (Linsenkäfer), Bruchus rufimanus (Pferdebohnenkäfer), Bruchus pisorum (Erbsenkäfer), Sitona lineatus (Linierter Blattrandkäfer), Otiorrhynchus sulcatus (Gefurchter Lappenrüßler), Otiorrhynchus ovatus (Erdbeerwurzelrüßler), Hylobies abietis (Großer Brauner Rüsselkäfer), Byctiscus betulae (Rebenstecher), Anthonomus pomorum (Apfelblütenstecher), Anthonomus grandis (Kapselkäfer), Ceuthorrhynchus assimilis (Kohlschotenrüßler), Ceuthorrhynchus napi (Großer Kohltriebrüßler), Sitophilus granaria (Kornkäfer), Anisandrus dispar (Ungleicher Hilzborkenkäfer), Ips typographus (Buchdrucker), Blastophagus piniperda (Gefurchter Waldgärtner);

aus der Ordnung der Zweiflügel (Diptera) beispielsweise Lycoria pectoralis, Mayetiola destructor (Hessenfliege), Dasineura brassicae (Kohlschoten-Gallmücke), Contarinia tritici (gelbe Weizen-Gallmücke), Haplodiplosis equestris (Sattelmücke), Tipula paludosa (Wiesenschnake), Tipula oleracea (Kohlschnake), dacus cucurbitae (Melonenfliege), Dacus oleae (Olivenfliege), Ceratitis capitata (Mittelmeer-Fruchtfliege), Rhagoletis cerasi (Kirschfruchtfliege), Rhagoletis pomonella (Apfelmade), Anastrepha ludens (Mexikanische Fruchtfliege), Oscinella frit (Fritfliege), Phorbia coarctata (Brachfliege), Phorbia antiqua (Zwiebelfliege), Phorbia brassicae (Kleine Kohlfliege), Pegomya hyoscyami (Rübenfliege), Anopheles maculipennis, Culex pipiens, Aedes aegypti (Gelbfiebermücke), Aedes vexans, Tabanus bovinus (Rinderbremse), Tipula paludosa (Wiesenschnake), Tipula oleracea (Kohlschnake), Musca domestica (Stubenfliege), Fannia canicularis (Kleine Stubenfliege), Muscina stabulans, Glossina morsitans (Tsetse-Fliege) Oestrus ocis, Chrysmya macellaria, Chrysomya hominivorax, Lucilia cuprina, Lucilia sericata, Hypoderma lineata;

aus der Ordnung der Hautflügler (Hymenoptera) beispielsweise Athalia rosae (Rübenblattwespe), Hoplocampa minuta (Pflaumensärewespe), Monomorium pharaonis (Pharaoameise), Solenopsis geminate (Feuerameise), atta sexdens (Blattschneiderameise);

aus der Ordnung der Wanzen (Heteroptera) beispielsweise Nezara viridula (Grüne Reiswanze), eurygaster integriceps (Asiatische Getreidewanze), Blissus leucopterus (Chinch bug), Dysdercus cingulatus (Kapok-Wanze), Dysdercus intermedius (Baumwollwanze), Piesma quadrata (Rübenwanze), Lygus pratensis (gemeine Wiesenwanze);

aus der Ordnung der Pflanzensauger (Homoptera) beispielsweise Perkinsiella saccharicida (Zuckerrohrzikade), Nilaparvata lugens (Braune Zikade), Empoasca fabae (Kartoffelzikade), Psylla mali (Apfelblattsauger), Psylla piri (Birnblattsauger), Trialeurodes vaporariorum (Weiße Fliege), Aphis fabae (Schwarze Bohnenlaus), Aphis pomi (Grüne Apfellaus), Aphis sambuci (Holunderblattlaus), Aphidula nastrutii (Kreuzdornblattlaus), Cerosipha gossypii (Gurkenblattlaus), Sappaphis mali (Rosige Apfellaus), Sappaphis mala (Mehlige Birnblattlaus), Dysphis radicola (Mehlige Apfelfalterlaus), Brachycaudus cardui (Große Pflaumenblattlaus), Brevicoryne brassicae (Kohlblattlaus), Phorodon humuli (Hopfenblattlaus), Rhopalomyzus ascalonicus (Zwiebellaus), Myzodes persicae (Grüne Pfirsichlaus), Myzus cerasi (Schwarze Sauerkirschenlaus), Dysaulacorthum pseudo-solani (Gefleckte Kartoffellaus), Acyrthosiphon onobrychis (Grüne Erbsenlaus), Macrosiphon rosae (Große Rosenblattlaus), Megoura viciae (Wickenlaus), Schizoneura lanuginosa (Birnenblattlaus), Pemphigus bursarius (Salatwurzellaus), Dreyfusia nordmannianae (Tannentrieblaus), Dreyfusia piceae (Weißtannenstammlaus), Adelges laricis (Rote Fichtengallenlaus), Viteus vitifolii (Reblaus);

aus der Ordnung der Termiten (Isoptera) beispielsweise Reticulitermes lucifugus, Calotermes flavicollis, Leucotermes flavipes, Termes natalensis;

aus der Ordnung der Geradflügler (Orthoptera) beispielsweise Forficula auricularia (Gemeiner Ohrwurm), Acheta domestica (Heimchen), Gryllotalpa gryllotalpa (Maulwurfsgrille), Tachycines asynamorus (Gewächshausschrecke), Locusta migratoria (Wanderheuschrecke), Stauronotus maroccanus (Marokkanische Wanderheuschrecke), Schistocerca peregrina (Wanderheuschrecke), Nomadacris septemfasciata (Wanderheuschrecke), Melanoplus spretus (Felsengebirgsheuschrecke), Melanoplus femur-rubrum (Rotbeinige Heuschrecke), Blatta orientalis (Küchenschabe), Blattella

germanica (Deutsche Schabe), Periplaneta americana (Amerikanische Schabe), Blabera gigantea (Riesenschabe).

Zur Klasse der Milben und Zecken (Arachnoidea) gehören beispielsweise Ixodes ricinus (Holzbock), Ornithodorus moubata, Amblyomma americanum, Dermacentor silvarum, Boophilus microplus, Tetranychus telarius, Tetranychus atlanticus, Tetranychus pacificus, Paratetranychus pilosus, Bryobia praetiosa.

Die erfindungsgemäßen Verbindungen können mit Erfolg im Pflanzenschutz sowie auf dem Hygiene-, Vorratsschutz- und Veterinärsektor als Schädlingsbekämpfungsmittel eingesetzt werden.

Die Wirkstoffe können als solche, in Form ihrer Formulierungen oder den daraus bereiteten Anwendungsmöglichkeiten, z. B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Äthanol, Propanol, Butanol, Chloroform, Tetrachlorkohlenstoff, Cyclohexanol, Cyclohexanon, Chlorbenzol, Isophoron, stark polare Lösungsmittel, z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholgykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylenoctylphenoläther, äthoxyliertes Isooctylphenol, Octylphenol, Nonylphenon, Alkylphenolpolyglykoläther, Tributylphenylpolyglykoläther, Alkylarylpolyätheralkohole, Isotridecylalkohol, Fettalkoholäthylenoxid-Kondensate, äthoxylieres Rizinusöl, Polyoxyäthylenalkyläther, äthoxyliertes Polyoxypropylen, Laurylalkoholpolyglykolätheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.-% Wirkstoff, vorzugsweise zwischen 0,5 und 90 Gew.-%.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit über 95 Gew.-% Wirkstoff oder sogar den Wirkstoff ohne Zusätze auszubringen.

Beispiele für Formulierungen sind:

I. 3 Gewichtsteile 2,2-Dimethyl-3-(2',2'-dichlorvinyl)-cyclopropancarbonsäure-(2'',2''-dimethyl-2',3''-dihydrobenzofuran-6''-methyl-(α-vinyl)-ester werden mit 97 Gewichtsteilen feinteiligem Kaolin innig vermischt. Man erhält auf diese Weise ein Stäubemittel, das 3 Gewichtsprozent des Wirkstoffs enthält.

II. 30 Gewichtsteile 2,2-Dimethyl-3-(2',2'-dichlorvinyl)-cyclopropancarbonsäure-(2'',2''-dimethyl-2'',3''-dihydrobenzofuran-6''-methyl)-ester werden mit einer Mischung aus 92 Gewichtsteilen pulverförmigem Kieselsäuregel und 8 Gewichtsteilen Paraffinöl, das auf die Oberfläche dieses

Kieselsäuregels gesprüht wurde innig vermischt. Man erhält auf diese Weise eine Aufbereitung des Wirkstoffs mit guter Haftfähigkeit.

III. 20 Gewichtsteile 2,2-Dimethyl-3-(2',2'-dichlorvinyl)-cyclopropancarbonsäure-[2'',2''-dimethyl-2'',3''-dihydrobenzofuran-6-methyl-(α-isopropyl)]-ester werden in einer Mischung gelöst, die aus 80 Gewichtsteilen Xylol, 10 Gewichtsteilen des Anlagerungsproduktes von 8 bis 10 Mol Äthylenoxid an 1 Mol Ölsäure-N-monoäthanolamid, 5 Gewichtsteilen Calciumsalz der Dodecylbenzolsulfonsäure und 5 Gewichtsteilen des Anlagerungsproduktes von 40 Mol Äthylenoxid an 1 Mol Ricinusöl besteht. Durch Ausgießen und feines verteilen der Lösung in 100 000 Gewichtsteilen Wasser erhält man eine wäßrige Dispersion, die 0,02 Gewichtsprozent des Wirkstoffs enthält.

Zu den Wirkstoffen können Öle verschiedenen Typs, Herbizide, Fungizide, andere Insektizide, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1 : 10 bis 10 : 1 zugemischt werden.

Beispielsweise können folgende Mittel zugemischt werden:

1,2-Dibrom-3-chlorpropan,
1,3-Dichlorpropen,
1,3-Dichlorpropen + 1,2-Dichlorpropan,
1,2-Dibrom-äthan,
2-sec.-Butyl-phenyl-N-methylcarbamat,
o-Chlorphenyl-N-methylcarbamat,
3-Isopropyl-5-methylphenyl-N-methylcarbamat,
o-Isopropoxyphenyl-N-methylcarbamat,
3,5-Dimethyl-4-methylmercapto-phenyl-N-methylcarbamat,
4-Dimethylamino-3,5-xylyl-N-methylcarbamat,
2-(1,3-dioxolan-2-yl)-phenyl-N-methylcarbamat,
1-Naphthal-N-methylcarbamat,
2,3-Dihydro-2,2-dimethyl-benzofuran-7-yl-N-methylcarbamat,
2,2-Dimethyl-1,3-benzodioxol-4-yl-N-methylcarbamat,
2-Dimethylamino-5,6-dimethyl-4-pyrimidinyl-dimethylcarbamat,
2-Methyl-2-(methylthio)-propionaldehyd-O-(methylcarbamoyl)-oxim,
S-Methyl-N-[(methylcarbamoyl)-oxy]-thio-acetimidat,
Methyl-N',N'-dimethyl-N-[(methylcarbamoyl)oxy]-1-thiooxamidat,
N-(2-Methyl-4-chlor-phenyl)-N',N'-dimethylformamidin,
Tetrachlorthiophen,
1-(2,6-Difluor-benzoyl)-3-(4-chlor-phenyl)-harnstoff,
O,O-Dimethyl-O-(p-nitrophenyl)-phosphorthioat,
O,O-Diäthyl-O-(p-nitrophenyl)-phosphorthioat,
O-Äthyl-O-(p-nitrophenyl)-phenyl-phosphonothioat,
O,O-Dimethyl-O-(3-methyl-4-nitrophenyl)-phosphorthioat,
O,O-Diäthyl-O-(2,4-dichlorphenyl)-phosphorthioat,
O-Äthyl-O-(2,4-dichlorphenyl)-phenyl-phosphonothioat,
O,O-Dimethyl-O-(2,4,5-trichlorphenyl)-phosphorthioat,
O-Äthyl-O-(2,4,5-trichlorphenyl)-äthyl-phosphonothioat,
O,O-Dimethyl-O-(4-brom-2,5-dichlorphenyl)-phosphorthioat,
O,O-Dimethyl-O-(2,5-dichlor-4-jodphenyl)-phosphorthioat,
O,O-Dimethyl-O-(3-methyl-4-methylthiophenyl)-phosphorthioat,
O-Äthyl-O-(3-methyl-4-methylthiophenyl)-isopropyl-phosphoramidat,
O,O-Diäthyl-O-[p-(methylsulfinyl)phenyl]-phosphorthioat,
O-Äthyl-S-phenyl-äthyl-phosphonodithioat,
O,O-Diäthyl-[2-chlor-1-(2,4-dichlorphenyl)-vinyl]-phosphat,
O-O-Dimethyl-[-2-chlor-1-(2,4,5-trichlorphenyl)]-vinyl-phosphat,
O,O-Dimethyl-S-(1'-phenyl)-äthylacetat-phosphordithioat,
Bis-(dimethylamino)-fluorphosphinoxid,
Octamethyl-pyrophosphoramid,
O,O,O,O-Tetraäthyldithio-pyrophosphat,
S-Chlormethyl-O,O-diäthyl-phosphordithioat,
O-Äthyl-S,S-dipropyl-phosphordithioat,
O,O-Dimethyl-O-2,2-dichlorvinyl-phosphat,
O,O-Dimethyl-1,2-dibrom-2,2-dichloräthylphosphat,
O,O-Dimethyl-2,2,2-trichlor-1-hydroxy-äthylphosphonat,
O,O-Dimethyl-S-[1,2-biscarbäthoxy-äthyl-(1)]-phosphordithioat,
O,O-Dimethyl-O-(1-methyl-2-carbmethoxy-vinyl)-phosphat,

O,O-Dimethyl-S-(N-methyl-carbamoyl-methyl)-phosphordithioat,
O,O-Dimethyl-S-(N-methyl-carbamoyl-methyl)-phosphorthioat,
O,O-Dimethyl-S-(N-methoxyäthyl-carbamoyl-methyl)-phosphordithioat,
O,O-Dimethyl-S-(N-formyl-N-methyl-carbamoylmethyl-phosphordithioat,
O,O-Dimethyl-O-[1-methyl-2-(methyl-carbamoyl)-vinyl]-phosphat,
O,O-Dimethyl-O-[(1-methyl-2-dimethylcarbamoyl)-vinyl]-phosphat,
O,O-Dimethyl-O-[(1-methyl-2-chlor-2-diäthylcarbamoyl)-vinyl]-phosphat,
O,O-Diäthyl-S-(äthylthio-methyl)-phosphordithioat,
O,O-Diäthyl-S-[(p-chlorphenylthio)-methyl]-phosphordithioat,
O,O-Dimethyl-S-(2-äthylthioäthyl)-phosphorthioat,
O,O-Dimethyl-S-(2-äthylthioäthyl)-phosphordithioat,
O,O-Dimethyl-S-(2-äthylsulfinyl-äthyl)-phosphorthioat,
O,O-Diäthyl-S-(2-äthylthio-äthyl)-phosphordithioat,
O,O-Diäthyl-S-(2-äthylsulfinyl-äthyl)-phosphorthioat,
O,O-Diäthyl-thiophosphoryliminophenyl-acetonitril,
O,O-Diäthyl-S-(2-chlor-1-phthalimidoäthyl)-phosphordithioat,
O,O-Diäthyl-S-[6-chlor-benzoxazolon-(2)-yl(3)]-methyldithiophosphat,
O,O-Dimethyl-S-[2-methoxy-1,3,4-thiadiazol-5-[4H]-onyl-(4)-methyl]-phosphordithioat,
O,O-Diäthyl-O-[3,5,6-trichlor-pyridyl-(2)]-phosphorthioat,
O,O-Diäthyl-O-(2-pyrazinyl)-phosphorthioat,
O,O-Diäthyl-O-[2-isopropyl-4-methyl-pyrimidinyl(6)]-phosphorthioat,
O,O-Diäthyl-O-[2-(diäthylamino)-6-methyl-4-pyrimidinyl]-thionophosphat,
O,O-Dimethyl-S-(4-oxo-1,2,3-benzotriazin-3-[4H]-yl-methyl)-phosphordithioat,
O,O-Dimethyl-S-[(4,6-diamino-1,3,5-triazin-2-yl)-methyl]-phosphordithioat,
O,O-Diäthyl-(1-phenyl-1,2,4-triazol-3-yl)-thionophosphat,
O,S-Dimethyl-phosphor-amido-thioat,
O,S-Dimethyl-N-acetyl-phosphoramidothioat,
γ-Hexachlorcyclohexan,
1,1-Di-(p-methoxyphenyl)-2,2,2-trichlor-äthan,
6,7,8,9,10,10-Hexachloro-1,5,5a,6,9,9a-hexahydro-6,9-methano-2,4,3-benzodioxa-thiepin-3-oxid,
Pyrethrine,
DL-2-Allyl-3-methyl-cyclopenten-(2)-on-(1)-yl-(4)-DL-cis,trans-chrysanthemat,
5-Benzyl-furyl-(3)-methyl-DL-cis,trans-chrysanthemat,
3-Phenoxybenzyl(±)-cis,trans-2,2-dimethyl-3-(2,2-dichlorvinyl)-cyclopropancarboxylat,
α-Cyano-3-phenoxybenzyl(±)-cis,trans-2,2-dimethyl-3-(2,2-dichlorvinyl) cyclopropancarboxylat,
(s)-α-Cyano-3-phenoxybenzyl-cis(1R,3R)-2,2-dimethyl-3-(2,2-dibromvinyl)-cyclopropancarboxylat,
3,4,5,6-Tetrahydrophthalimidoäthyl-DL-cis,trans-chrysanthemat,
2-Methyl-5-(2-propinyl)-3-furylmethyl-chrysanthemat,
α-Cyano-3-phenoxybenzyl-α-isopropyl-4-chlorphenylacetat.

Mit besonderem Vorteil werden Verbindungen der Formel (I) auch mit Substanzen kombiniert, welche einen synergistischen oder verstärkenden Effekt auf Pyrethroide ausüben. Beispiele solcher Verbindungen sind u. a.

Piperonylbutoxid, Propinyläther, Propinyloxime, Propinylcarbamate und Propinylphosphonate, 2-(3,4-Methylendioxyphenoxy)-3,6,9-trioxaundecan, S,S,S-Tributylphosphorotrithioate, 1,2-Methylendioxy-4-(2-(octylsulfonyl)-propyl)-benzol, 1-n-Dodecylimidazol, 1-(1,5,9-Trimethyldecyl)-imidazol, 1-[2-Chlor-2-(4-fluorphenyl)-äthyl]-1,2,4-triazol, 1-(2-Phenyläthyl)-1,2,4-triazol, 1-(2-Chlor-2-phenyl-äthyl)-1,2,4-triazol, 1-(3-Phenyl-n-propyl)-1,2,4-triazol.

Die folgenden Beispiele belegen die biologische Wirkung der neuen Ester. Vergleichssubstanz ist 2,2-Dimethyl-3-(2′,2′-dimethylvinyl)-cyclopropancarbonsäure-(2,2-dimethyl-2,3-dihydrobenzofuran-6-methyl)-ester (DE-A-2 108 932). Die Numerierung der Wirkstoffe entspricht der vorstehenden Wirkstofftabelle.

## Beispiel A

### Kontaktwirkung auf Stubenfliegen (Musca domestica); Dauerkontakt

Beide Teile einer Petrischale von 10 cm Durchmesser werden mit insgesamt 2 ml der acetonischen Wirkstofflösung ausgekleidet. Nach dem Verdunsten des Lösungsmittels (ca. 30 Minuten) bringt man je 10 Fliegen in die Schalen. Die Mortalitätsrate wird nach 4 Stunden festgestellt.

| Wirkstoff Nr. | Wirkstoffmenge pro Petrischale [mg] | Mortalitätsrate [%] |
|---|---|---|
| 1 | 0,005 | 100 |
| 2 | 0,2 | 100 |
| 3 | 0,01 | 100 |
| 4 | 0,005 | 100 |

## Beispiel B

### Kontaktwirkung auf Stubenfliegen (Musca domestica); Applikationstest

4 Tage alte Imagines erhalten in leichter $CO_2$-Narkose 1 µl der acetonischen Lösung des Wirkstoffs auf das ventrale Abdomen appliziert. Hierzu wird eine Mikrometerspritze verwendet. Je 20 Versuchstiere mit gleicher Behandlung bringt man dann in einen Folienbeutel (Volumen ca. 500 ml).

Nach 4 Stunden zählt man die Tiere in Rückenlage aus und ermittelt graphisch die LD 50.

| Wirkstoff Nr. | LD 50 |
|---|---|
| 1 | 0,017 µg/Fliege |
| 3 | 0,03 µg/Fliege |
| 4 | 0,022 µg/Fliege |
| Vergleichssubstanz | 0,12 µg/Fliege |

## Beispiel C

### Fraß- und Kontaktwirkung auf Raupen der Kohlschabe (Plutella maculipennis)

Blätter von jungen Kohlpflanzen werden 3 Sekunden lang in die wäßrige Wirkstoffemulsion getaucht und nach kurzem Abtropfen auf einen angefeuchteten Filter in einer Petrischale gelegt. Das Blatt wird darauf mit 10 Raupen des 4. Stadiums belegt.

Nach 48 Stunden beurteilt man die Wirkung.

| Wirkstoff Nr. | Konzentration der Wirkstoffemulsion [Gew.%] | Mortalitätsrate [%] |
|---|---|---|
| 1 | 0,005 | 100 |
| | 0,002 | ca. 80 |
| 2 | 0,02 | 100 |
| 3 | 0,02 | 100 |
| | 0,01 | ca. 80 |
| 4 | 0,004 | 100 |

Beispiel D

Kontaktwirkung auf Moskito-Larven (Aedes aegypti)

200 ml Leitungswasser werden mit der Wirkstoffaufbereitung versetzt und darauf mit 30 bis 40 Moskito-Larven im 4. Larvenstadium besetzt. Die Versuchstemperatur beträgt 20° C. Nach 25 Stunden wird die Wirkung ermittelt.

| Wirkstoff Nr. | Konzentration der Wirkstoffaufbereitung [ppm] | Mortalitätsrate [%] |
|---|---|---|
| 1 | 0,01 | 100 |
| 3 | 0,02 | 100 |
| 4 | 0,02 | 100 |
| Vergleichssubstanz | 0,04 | ca. 80 |

Beispiel E

Kontaktwirkung auf Zecken (Ornithodorus moubata)

Geprüft wird mit Zecken im 3. Larvenstadium. Dazu taucht man die Tiere, die sich in einem Papierbeutel befinden, für 3 Sekunden in die Prüfemulsion. Die Beutel werden frei aufgehängt. Nach 48 Stunden wird die Wirkung auf die Zecken beurteilt.

| Wirkstoff Nr. | Wirkstoffkonzentration der Prüfemulsion [Gew.%] | Mortalitätsrate [%] |
|---|---|---|
| 1 | 5 | 100 |
| 2 | 20 | 100 |
| 3 | 20 | 100 |
| 4 | 20 | 100 |

**Patentansprüche**

1. Substituierte 2,3-Dihydrobenzofurylmethyl-cyclopropancarbonsäureester der Formel (I)

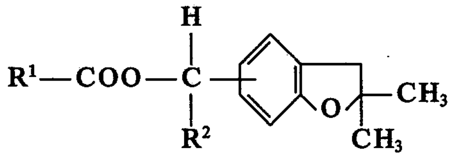 (I)

in der

$R^1$ für den 3-(2,2-Dichlorvinyl)- oder den 3-(2,2-Dibromvinyl)-2,2-dimethyl-cyclopropylrest und
$R^2$ für Wasserstoff, Alkyl oder Alkenyl mit jeweils bis zu 5 Kohlenstoffatomen stehen.

2. Schädlingsbekämpfungsmittel, gekennzeichnet durch einen Gehalt an Verbindungen gemäß Anpsruch 1.

3. Schädlingsbekämpfungsmittel, enthaltend einen festen oder flüssigen Trägerstoff und mindestens eine Verbindung gemäß Anspruch 1.

4. Verfahren zur Bekämpfung von Schädlingen, dadurch gekennzeichnet, daß man Verbindungen gemäß Anspruch 1 auf die genannten Schädlinge bzw. deren Lebensraum einwirken läßt.

5. Verfahren zur Herstellung von Schädlingsbekämpfungsmitteln, dadurch gekennzeichnet, daß man Verbindungen gemäß Anspruch 1 mit Streckmitteln und/oder oberflächenaktiven Mitteln mischt.

6. Verfahren zur Herstellung von substituierten 2,3-Dihydro-benzofurylmethyl-cyclopropancarbonsäureestern der Formel I gemäß Anspruch 1, dadurch gekennzeichnet, daß man Säurehalogenide der Formel (II)

$$R^1-CO-Hal \qquad (II)$$

in der $R^1$ die in Anspruch 1 genannten Bedeutungen hat und Hal für Halogen steht, in Gegenwart eines säurebindenden Mittels mit Verbindungen der Formel (III)

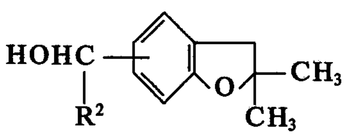 (III)

in der $R^2$ die in Anspruch 1 genannten Bedeutungen hat, umsetzt.

**Claims**

1. Substituted 2,3-dihydrobenzofurylmethyl-cyclopropanecarboxylic acid esters of the formula (I)

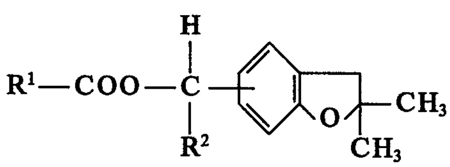 (I)

where

$R^1$ is a 3-(2,2-dichlorovinyl)- or 3-(2,2-dibromovinyl)-2,2-dimethylcyclopropyl radical, and
$R^2$ is hydrogen, alkyl or alkenyl, each of up to 5 carbon atoms.

2. A pesticide containing a compound as claimed in claim 1.

3. A pesticide containing a solid or liquid carrier and at least one compound as claimed in claim 1.

4. A process for combating pests, wherein a compound as claimed in claim 1 is allowed to act on the pests or their habitat.

5. A process for the manufacture of pesticides, wherein a compound as claimed in claim 1 is mixes with extenders and/or surfactants.

6. A process for manufacturing substituted 2,3-dihydrobenzofurylmethyl-cyclopropane carboxylic acid esters of the formula I as claimed in claim 1, wherein an acid halide of the formula (II)

$$R^1-CO-Hal \qquad (II)$$

where $R^1$ has the meanings given in claim 1 and Hal is halogen, is reacted with a compound of the formula (III)

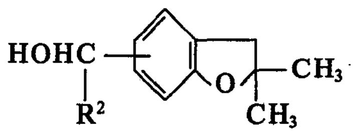

(III)

where $R^2$ has the meanings given in claim 1, in the presence of an acid-binding agent.

**Revendications** .

1. Esters 2-3-dihydrobenzofurylméthyliques substitués de l'acide cyclopropanecarboxylique, de formule (I)

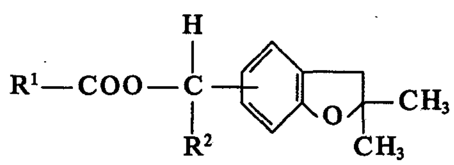

(I)

dans laquelle

$R^1$ représente le reste 3-(2,2-dichlorovinyl)- ou le 3-(2,2-dibromovinyl)-2,2-diméthylcyclopropyle et
$R^2$ représente hydrogène, alkyle ou alcényle ayant chacun jusqu'à 5 atomes de carbone.

2. Agent de lutte contre les parasites, caractérisé par le fait qu'il contient des composés selon la revendication 1.
3. Agent de lutte contre les parasites contenant un véhicule solide ou liquide et au moins un composé selon la revendication 1.
4. Procédé de lutte contre les parasites, caractérisé par le fait qu'on fait agir des composés selon la revendication 1 sur les parasites indiqués ou leur biotope.
5. Procédé de préparation d'agent de lutte contre les parasites, caractérisé par le fait qu'on mélange des composés selon la revendication 1 avec un diluant et/ou des agents tensio-actifs.
6. Procédé de préparation d'esters 2-3-dihydrobenzofurylméthyliques substitués de l'acide cyclopropanecarboxylique, de formule I selon la revendication 1, caractérisé par le fait qu'on fait réagir des halogénures d'acides de formule (II)

$$R^1-CO-Hal \qquad (II)$$

dans laquelle $R^1$ a la signification indiquée dans la revendication 1 et Hal représente halogène, en présence d'un agent liant les acides, avec des compos'es de formule (III)

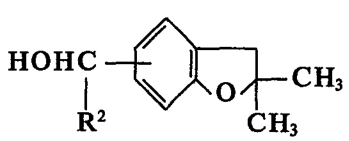

(III)

dans laquelle $R^2$ a la signification indiquée dans la revendication 1.